# EUROPEAN PATENT SPECIFICATION

(11) **EP 0 614 485 B1**
(45) Date of publication and mention of the grant of the patent: **17.07.2002**
(21) Application number: 92923129.8
(22) Date of filing: 22.10.1992
(51) Int. Cl.: C12N 5/08

(54) **METHODS FOR SELECTIVELY EXPANDING CD34 POSITIVE CELLS**
VERFAHREN ZUR SELEKTIVEN VERMEHRUNG VON CD34 POSITIVEN ZELLEN
METHODES POUR LA MULTIPLICATION SELECTIVE DE CELLULES CD34 POSITIVES

(30) Priority: 23.10.1991 US 780488
(43) Date of publication of application: 14.09.1994
(73) Proprietor: Nexell Therapeutics Inc., Irvine, CA 92618-1605 (US)
(72) Inventor: HEIMFELD, Shelly, Woodinville, WA 98072 (US); BERENSON, Ronald, J., Mercer Island, WA 98040 (US); FEI, RuiGao, Seattle, WA 98115 (US); GOFFE, Randal, A., Bothell, WA 98021 (US); PETERSON, Dale, R., Bothell, WA 98021 (US); PORTER, Christopher, H., Woodinville, WA 98072 (US)
(74) Representative: Gowshall, Jonathan Vallance
(86) International application number: US9209019
(87) International publication number: WO9308268

(56) References cited:
- EP-A- 0 341 966
- EP-A- 0 451 611
- EP-A- 0 455 482
- WO-A-92/01039

## Description

### Cross-Reference to Related Application

This application is a continuation-in-part of pending U.S. Application Serial No. 07/513,543, which was filed April 23, 1990 (US Patent No. 5635387).

### Technical Field

The present invention relates generally to cells which make up bone marrow, and more specifically, to methods which may be utilized to selectively expand the number of CD34 positive cells.

### Background of the Invention

Cancer accounts for over one-fifth of the total mortality in the United States, and is the second leading cause of death. The leading types of cancer in men are lung, prostate, and colorectal cancer, and for women, breast, lung and colorectal cancer. Currently, most cancers are treated with a combination of therapies involving surgery, and chemotherapy and/or radiation therapy.

One difficulty with chemotherapy and radiotherapy, however, is that it also destroys an individual's immune system, as well as stem cells, the progenitor cells of the immune system. In order to reconstitute the immune system, a patient undergoes bone marrow transplantation with generally either allogenic or autologous bone marrow. Many individuals, however, die because their own bone marrow is involved with the cancer, or a histocompatible donor cannot be found.

One method that has been suggested to overcome this difficulty is the use of long-term marrow cultures which prolong the life of engrafting cells. For example, Dexter *et al.* (*J. Cell Phys. 91*:335, 1976) describes conditions for proliferating stem cells *in vitro* (hereinafter referred to as "Dexter cultures"). Briefly, this method involves the establishment of an adherent monolayer of stromal cells, which supports the viability of stem and early progenitor cells. Dexter cultures, however, are ultimately disadvantageous because they only slow the death rate of stem and progenitor cells, and do not provide the desired result of increasing the number of such cells.

The present invention provides methods for selectively expanding CD34⁺ cells. These methods overcome disadvantages of prior methods, and further provide other related advantages.

### Summary of the Invention

Briefly stated, the present invention is directed toward methods for selectively expanding CD34⁺ cells. Within one aspect of the present invention, a method for selectively expanding CD34⁺ cells is provided, comprising the steps of (a) separating stem cells from other cells, and (b) incubating the separated CD34 positive cells in a selected medium containing stem cell growth factor, Mast cell Growth Factor, Interleukin 1, Interleukin 3, Interleukin 4, Interleukin 6, Interleukin 7, G-CSF, GM-CSF, M-CSF, TGF-β, TNF-α, α-JNF, FGF, PDGF, JGF-1 or JGF-2, such that the CD34 positive cells are selectively increased in number. Within one embodiment the CD34 positive cells are periodically spearated from mature cells.

Within other embodiments, the CD34⁺ cells are separated on an affinity column, or by Flow Cytometry. Within further embodiments, the separated CD34⁺ cells are incubated in a petri dish, in a sterile bag, or in hollow fibers.

Within another embodiment of the present invention, a method for selectively expanding CD34⁺ cells is provided, comprising the steps of (a) separating CD34⁺ cells from other cells on an affinity column, and (b) incubating the separated CD34⁺ cells in a sterile bag with a medium containing Stem Cell Growth Factor.

These and other aspects of the present invention will become evident upon reference to the following detailed description and attached drawings.

### Brief Description of the Drawings

Figure 1 is a graph which illustrates the effect of separating out stem cells on stem cell expansion, as determined by the increase in total cell number.
Figure 2 is a graph which illustrates the effect of separating out stem cells on stem cell expansion, as determined by the increase in CFCs.
Figure 3 is a graph which illustrates the effect of various growth factors on total cell number.
Figure 4 is a graph which illustrates the effect of various growth factors on the number of CFCs.

### Detailed Description of the Invention

The present invention provides methods for selectively expanding CD34⁺ cells. Within the context of the present invention, the term CD34⁺ cell" refers to totipotent hematopoietic stem cells as well as early progenitor cells such as colony-forming cells (CFCs). These cells may be differentiated from other cells based upon the presence of CD 34 receptors.

As noted above, within one aspect of the present invention, methods are provided for selectively expanding CD34⁺ cells, comprising the steps of (a) separating CD34⁺ cells from other cells, and (b) incubating the separated CD34⁺ cells with a selected media which is capable of expanding the stem cells.

Utilizing the devices and methods which are described in greater detail below, stem cells may be separated from various blood products, including for example, peripheral blood and whole bone marrow. For purposes of the present invention, stem cells are considered to be separated if at least 20% of the separated cells are CD 34 positive cells. Preferably, the CD 34 positive cells should be separated to provide greater than 90% purity. In addition, it may be desirable to keep the total numbers of platelets, granulocytes, and red cells as low as possible in order to prevent clumping and the release of degradative enzymes which decrease engrafting cell recovery and viability. More specifically, it may be desirable that the stem cells contain less than about 1% platelets, less than 50% and preferably less than about 25% granulocytes, and less than 10% and preferably less than about 1% red cells.

Separation of CD34⁺ cells may be accomplished through use of a ligand which specifically recognizes antigens on these cells. For example, antibodies which specifically recognize the CD 34 antigen may be utilized in the devices and methods described below in order to separate stem cells. Representative examples of antibodies which specifically recognize the CD 34 antigen include My-10 and HPCA2 (Becton-Dickinson, Mountain View Calif.) and 12.8 (CellPro®, Bothell, Wash.).

Various methods and devices may be utilized to separate CD34⁺ cells, including the use of magnetic beads, panning, and flow cytometry (Fluorescence Activated Cell Sorting "FACS") (*see*, *for example*, U.S. Patent Nos. 4,714,680 and 4,965,204). Particularly preferred methods and devices are immunoaffinity columns such as those which are described in WO-A-91 16116, entitled "Immunoselection Device and Method". Briefly, this application describes methods and devices for isolating or separating target particles such as stem cells, from a mixture of non-target and target particles. Included within this application is a discussion of devices and methods wherein target particles are separated in a direct method by passing the particles through a column containing a bed of low nonspecific binding porous material which has a ligand capable of specifically binding to the target particles. Within one aspect of the application, a device is provided which generally comprises (a) a column having a proximal end with an inlet port through which fluid may enter the column and a distal end with an outlet port through which fluid may exit the column, (b) a bed of low nonspecific binding porous material within the column, the porous material having a biotin adsorbing group immobilized on the surface thereof, wherein the pores of the porous material are of a size sufficient to allow the biotin adsorbing group to enter into the pores, but not so large as to allow collapse of the bed, and wherein the interstitial spaces of the bed are of a size sufficient to allow the particles to flow through the bed. The device may further comprise a means, located within the column, for agitating the porous material upon the application of an external force, such that bound target particles are released from the porous material. Within other aspects of this application the target particles are separated by either a one-step or two-step method utilizing avidin and biotin. It should also be noted, however, that for purposes of the present invention other materials may be utilized within the immunoaffinity column, including for example non-porous materials.

A particularly preferred immunoaffinity column is a "cell separator" including a column assembly for separating target cells from a sample fluid, the column assembly including a column, a sample fluid supply bag and a fluid collection bag wherein the column is provided for receiving the sample fluid from the sample fluid supply bag and for separating the target cells from the sample fluid and retaining the target cells, and wherein the fluid collection bag is provided for receiving the target cells after being released from the column, said cell separator, comprising an agitation means for agitating the contents of the column to assist in releasing the sample cells retained in the column, the agitation means being responsive to a drive signal for varying amounts of agitation of the contents of the column to vary the rate at which the sample cells are released, column sensor means for providing a column signal indicative of the optical density of fluid flowing out of the column and into the fluid collection bag, a column valve means response to a column valve control signal for selectively enabling the fluid coming out of the column to flow into the fluid collection bag, and a data processor means for controlling the operation of the cell separator, the data processor means being responsive to the column signal for providing the drive signal and the column valve control signal to prevent inadequate concentrations of the target cells from being collected. One embodiment of this disclosure is the CEPRATE LC™ cell separation system which is available from CellPro® (Bothell, Wash.).

The separated CD34 positive cells are then incubated in a selected medium such that the stem cells are selectively expanded. Within the context of the present invention, CD34⁺ cells are "selectively expanded" if CD34⁺ cells increase in number more than other cell types as a whole. Briefly, the media should comprise a nutrient base which maintains cell viability, as well as a factor which expands the number of CD84⁺ cells. Representative examples of nutrient bases include RPMI, TC 199, or Iscoves DMEM, which have been supplemented with a protein such as fetal bovine serum. Alternatively, the nutrient base may be a defined medium such as Ex Vivo. Various components may be utilized in order to selectively expand CD34⁺ cells. Representative factors include Interleukin-1, Interleukin-3, Interleukin-4, Interleukin-6, Interleukin-7, SCF, GM-CSF, G-CSF, M-CSF, TGF-β, TNF-α, α-INF, FGF, PDGF, IGF-1, and IGF-2. Particularly preferred factors include Stem Cell Growth Factor (Amgen, Thousand Oaks, Calif.), Interleukin-3, Granulocyte-Macrophage Colony-Stimulating Factor (Immunex, Seattle, Wash.), Granulocyte Colony-Stimulating Factor, Interleukin-6 (Amgen, Thousand Oaks, California), and Mast Cell Growth Factor (Immunex, Seattle, Wash.).

One may readily determine whether a medium selectively expands CD34⁺ cells by purifying CD34⁺ cells as described above, and incubating them as described below with a selected medium. The number of CD34⁺ cells and other cells are counted prior and subsequent to incubation. As noted above, CD34⁺ cells have been selectively expanded if they increase in number, while the other cells do not. Total numbers of cells may be counted by standard techniques (*e.g.*, hemacytometer), and numbers of CD34⁺ cells may be counted by flow cytometry (*i.e.*, cells labeled with fluorescently conjugated anti-CD34 antibody may be counted by FACS) or as described below in Example 3.

The separated CD34⁺ cells may be incubated in a variety of containers. Particularly preferred containers include: petri dishes (Corning Glass Works, Corning, N.Y.), 6-well plates (Costar), gas permeable sterile bags such as Stericell (Terumo, Elkton, Md.), and Lifecell (Baxter, Deerfield, Ill.), and hollow fibers such as CellPhar2 (Unisyn Fibertec, San Diego, Calif.), Accusys (Endotronics, Minneapolis, Minn.), or Vitafiber (Amicon, Danvers, Mass.). Preferably, the cells should be incubated in an atmosphere of 5% to 10% CO₂, and at a temperature of about 37°C.

Within another embodiment of the invention, a method is provided for selectively expanding CD34⁺ cells, comprising the steps of (a) periodically separating CD34⁺ cells from mature cells, and (b) incubating the separated CD34⁺ cells in a selected medium such that the CD34⁺ cells are selectively expanded. Briefly, mature cells (which include not only terminally differentiated blood cells, but cells of an intermediate lineage) are believed to inhibit the expansion and differentiation of stem cells via a feedback control mechanism. Removal of mature cells from a culture thus permits expansion of the stem cells to many times their original numbers. Within the context of the present invention, periodically separating means removal of mature cells at least every 10 days.

Various methods may be utilized in order to periodically separate CD34⁺ cells. For example, within one embodiment, cells are separated on an affinity column as described above, incubated in a selected medium utilizing any of the containers discussed above, and then subsequently reseparated in order to separate the CD34⁺ cells from the newly differentiated mature cells.

CD34 positive cells may be continuously separated while being perfused with a selected medium such that the CD34⁺ cells are selectively expanded. Briefly, this may be accomplished by continuously perfusing the selected media through a separation device (such as an affinity column as discussed above). CD34⁺ cells are retained in the separation device while mature cells (which do not have CD 34 antigens) pass through the device. As CD34⁺ cells grow and expand in number, the new CD34⁺ cells similarly adhere to the separation device, while newly differentiated cells with no CD 34 antigens pass through the device.

CD34 positive cells may also be continuously separated in a perfusion device by passing beads through the perfusion chamber which have been coated with immobilized ligands to surface antigens found on mature cells, so that the mature cells are bound and carried out of the perfusion chamber by the beads.

The following examples are offered by way of illustration, and not by way of limitation.

### EXAMPLES

### EXAMPLE 1

### Preparation of an Avidinated Biogel

### A. CARBOXYLATION OF A POLYACRYLAMIDE GEL

Seventeen grams of dry Biogel P-60™ (50-100 mesh (wet), coarse beads) (BIORAD, Catalog No. 150, 1630, Richmond, Calif.) are added to 1.5 1 of 0.5 M NaHCO₃/0.5 M Na₂CO₃. The pH is adjusted to 10.5 with NaOH and carefully stirred with a mixer (RZR1, Carfamo, Wiarton, Ontario, Canada) so as not to damage the beads for approximately 20 to 30 minutes. The mixture is then placed in a 60°C water bath. After the mixture reached a temperature of 60°C, it is incubated for an additional 2 hours (at 60°C) with occasional stirring. The mixture is then removed from the water bath, and placed in an ice bath to bring the mixture temperature down to room temperature.

The beads are washed several times with distilled or deionized water, followed by several washings with PBS using a coarse glass filter connected to a vacuum. The carboxylated gel may be stored in PBS at 4°C, and is stable for up to one year if sterilized or stored with a preservative.

### B. AVIDIN CONJUGATION OF CARBOXYLATED BIOGEL

PBS is first removed from a measured amount of carboxylated Biogel by filtering with a coarse glass filter connected to a vacuum. The gel is then equilibrated in distilled or deionized water for 15 to 30 minutes. Equilibration in water causes an expansion of the gel to a volume of about 4 times its previously measured amount. The gel is resuspended in 10 ml of distilled or deionized water for each ml of gel (as originally measured in PBS).

Thirty mg of 1-ethyl-3-(3-dimethylaminopropyl) carbodiimide (EDC-HCl) (Sigma Chemical Co., Catalog No. E7750, St. Louis, Mo.) is added for each ml of gel as originally measured. The pH is rapidly adjusted to 5.5 by dropwise addition of HCl. Care is taken to maintain the pH at 5.5; pHs of less than 5.0 or greater than 6.0 result in significantly less activation of the Biogel. The mixture is stirred for five minutes.

Avidin (International Enzymes, Inc., Fallbrook, Calif.) is dissolved at a concentration of between 10 and 100 mg/ml in deionized water. Next, 1 mg of avidin is rapidly added for each ml of gel (as originally measured in PBS). The mixture is stirred for 1.5 hours. Next, 2 M glycine is added to give a final concentration of 0.2 M glycine in the mixture, and stirred for an additional 1 hour.

The gel is washed with several volumes of PBS using a coarse glass filter and vacuum, and stored in PBS at 4°C. The gel is stable for approximately one year.

### EXAMPLE 2

### Isolation of Engrafting Cells

### A. PREPARING THE BUFFY COAT CELLS

A sample of bone marrow is centrifuged at 240 x g for 15 minutes. The plasma is removed (and is retained for later use), and the remaining buffy coat cells are centrifuged once more at 240 x g for 15 minutes in order to remove red blood cells. The buffy coat cells are washed twice with RPMI by centrifugation at 180 x g for 10 minutes. The cells are then resuspended to a final concentration of 1 x 10⁸ white cells/ml in RPMI plus 1% BSA.

### B. INCUBATION OF BUFFY COAT CELLS WITH ANTIBODY

The suspension of buffy coat cells is incubated with a final concentration of 20 µg/ml biotinylated anti-CD 34 antibody (CellPro®, Bothell, Wash.) at room temperature for 25 minutes. The antibody-cell mixture is then washed twice with PBS by centrifugation at 180 x g for 10 minutes. The cells are then resuspended at a concentration of 1 x 10⁸ white cells/ml in PBS.

### C. COLUMN OPERATION AND RESULTS

A CEPRATE LC™ (CellPro®, Bothell, Wash.) separating system was utilized essentially according to the manufacturer's instructions. Briefly, the instrument was set up, the tubing connected, reagent's were loaded, and the process run was begun with the antibody treated cells. The cells were pumped through the column, the column was washed with PBS, then the adsorbed cells were released via the magnetically driven impeller. The adsorbed cells were accumulated in a collection bag.

### D. RESULTS

Ten billion bone marrow cells were passed through the column; 200 million of the cells were bound to the column and were recovered in the collection bag. Viability of the collected cells was 91% as measured by trypan blue exclusion. The collected cells were 75% CD 34⁺ as measured by FACS analysis.

### EXAMPLE 3

### Determination of CFC Viability and Recovery

One ml per 35 mm plate of Iscove's Methylcellulose (Terry Fox Laboratories, Vancouver, British Columbia, Canada) supplemented with 2 mM L-glutamine and 50 ng/ml gentamicin was warmed to 37°C. Cells were plated in triplicate at 3-fold dilutions to improve the accuracy of the assay. The highest number of cells plated was 10⁵/plate except for column-purified cells which were plated at 3 x 10³ and less. The cells were spread evenly over the surface of each plate and then incubated in a humidified incubator at 37°C with 5% CO₂ in air for 10 to 14 days. Colonies were counted if they contained more than 50 cells and scored as CFU-GM, BFU-E, or other (*e.g.,* CFU-GEMM). The number of various types of colonies were summed to give the total number of colony-forming cells (CFCs).

### EXAMPLE 4

### Stem Cell Expansion

### A. COMPARISON OF THE EXPANSION OF SEPARATED STEM CELLS VERSUS STEM CELLS IN WHOLE MARROW

Stem Cells which were purified as described above in Example 2 were grown in a solution containing RPMI 1640, 10% Fetal Bovine Serum HYCLONE®, Logan, Utah), 50 ng/ml Stem Cell Growth Factor, 50 ng/ml Interleukin-3, 20 ng/ml Granulocyte-Macrophage Colony-Stimulating Factor, and 20 ng/ml Granulocyte Colony-Stimulating Factor. The cells were plated at 10⁶ per plate in 1 ml of media. On days 7, 14, and 21 cells were removed and replated for CFC assays as described in Example 3. Viable cells were counted by hemacytometer using trypan blue.

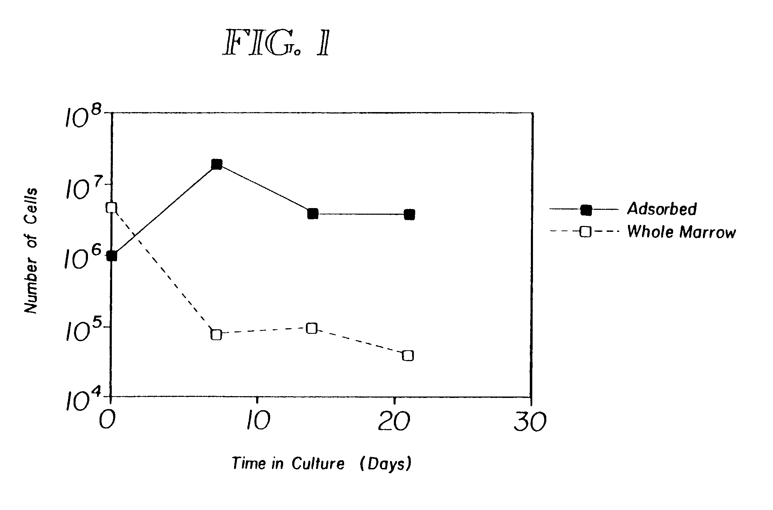

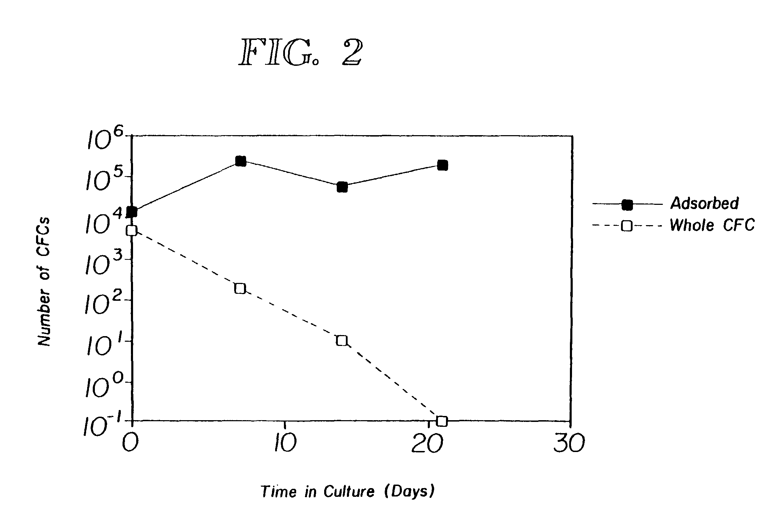

As illustrated by total cell count in Figure 1, and the number of CFCs in Figure 2, stem cell separation prior to culturing the cells dramatically improved stem cell growth and expansion.

### B. FACTORS WHICH CAUSE STEM CELL EXPANSION

In order to determine what growth factors are useful for expanding stem cells, the following assay was undertaken. Briefly, stem cells which were purified as described above, were plated at a density of 10⁶ cells per plate in Corning 35 mm plates. The cells were fed a solution containing RPMI 1640 supplemented with 10% fetal bovine serum, and various combinations of the following growth factors: (1) 50 ng/ml Stem Cell Growth Factor (Amgen, Thousand Oaks, Calif.), (2) 50 ng/ml Interleukin-3, (3) 20 ng/ml Granulocyte-Macrophage Colony-Stimulating Factor (Immunex, Seattle, Wash.), and (4) Granulocyte Colony-Stimulating Factor (Genzyme, Cambridge, Mass.).

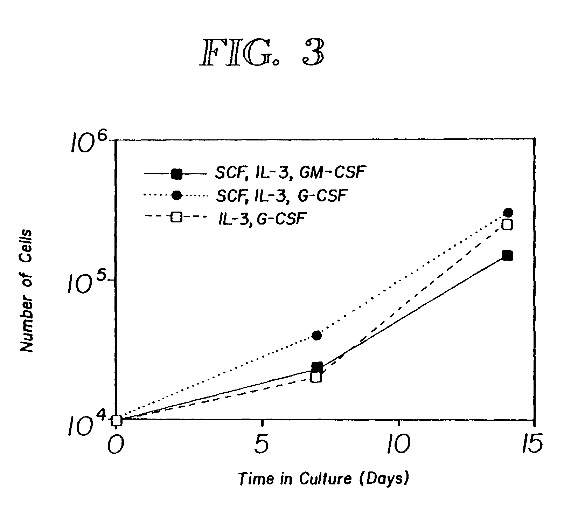

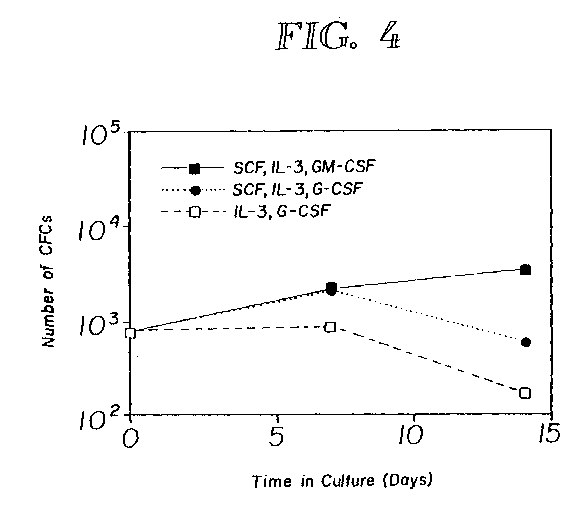

As illustrated in Figures 3 and 4, both media containing SCF expanded stem cells (as determined by the increase in CFC number).

From the foregoing, it will be appreciated that, although specific embodiments of the invention have been described herein for purposes of illustration, various modifications may be made without deviating from the spirit and scope of the invention. Accordingly, the invention is not limited except as by the appended claims.

## Claims

1. A method for selectively expanding CD34 positive cells, comprising:
(a) separating CD34 positive cells from other cells; and
(b) incubating the separated CD34 positive cells in a selected medium containing stem cell growth factor, Mast Cell Growth Factor Interleukin 1, Interleukin 3, Interleukin 4, Interleukin 6, Interleukin 7, G-CSF, GM-CSF, M-CSF, TGF-β, TNF-α, α-INF, FGF, PDGF, IGF-1 or IGF-2, such that the CD34 positive cells are selectively increased in number.

2. A method, according to Claim 1, for selectively expanding CD34 positive cells comprising:
(a) periodically separating CD34 positive cells from mature cells; and
(b) incubating the separated CD34 positive cells in said selected medium such that the CD34 positive cells are selectively increased in number.

3. The method of Claim 1 wherein said selected medium contains Stem Cell Growth Factor.

4. The method of Claim 1, wherein said selected medium contains Interleukin-3.

5. The method of Claim 1 wherein said selected medium contains Granulocyte-Macrophage Colony-Stimulating Factor.

6. The method of Claim 1 wherein said selected medium contains Granulocyte Colony-Stimulating Factor.

7. The method of Claim 1 wherein said selected medium contains Interleukin-6.

8. The method of Claim 1 wherein said selected medium contains Mast Cell Growth Factor.

9. The method of Claim 1 wherein said selected medium contains Interleukin-1.

10. The method of any one of Claims 1 to 9 wherein said CD34 positive cells are separated by immunoselection.

11. The method of Claim 10 wherein said CD34 positive cells are separated on an affinity column.

12. The method of Claim 10 wherein said CD34 positive cells are separated by flow cytometry.

13. The method of any one of Claims 1 to 12 wherein said separated CD34 positive cells are incubated in a petri dish.

14. The method of any one of Claims 1 to 13 wherein said separated CD34 positive cells are incubating in a sterile bag.

15. The method of any one of Claims 1 to 13 wherein said separated CD34 positive cells are incubated in hollow fibers.

16. A method according to Claim 1 selectively expanding CD34 positive cells, comprising:
(a) separating CD34 positive cells from the other cells on an affinity column; and
(b) incubating the separated CD34 positive cells in a sterile bag with a medium containing Stem Cell Growth Factor.

## Patentansprüche

1. Verfahren zur selektiven Expansion von CD34 positiven Zellen, umfassend:
(a) Abtrennen von CD34 positiven Zellen von anderen Zellen; und
(b) Inkubieren der abgetrennten CD34 positiven Zellen in einem ausgewählten Medium, das Stammzellwachstumsfaktor, Mastzellwachstumsfaktor, Interleukin 1, Interleukin 3, Interleukin 4, Interleukin 6, Interleukin 7, G-CSF, GM-CSF, M-CSF, TGF-β, TNF-α, α-INF, FGF, PDGF, IGF-1 oder IGF-2 enthält, so daß die CD34 positiven Zellen selektiv in ihrer Zahl zunehmen.

2. Verfahren, zur selektiven Expansion von CD34 positiven Zellen nach Anspruch 1, umfassend:
(a) periodisches Abtrennen von CD34 positiven Zellen von reifen Zellen; und
(b) Inkubieren der abgetrennten CD34 positiven Zellen in dem ausgewählten Medium, so daß die CD34 positiven Zellen selektiv in ihrer Zahl zunehmen.

3. Verfahren nach Anspruch 1, wobei das ausgewählte Medium Stammzellwachstumsfaktor enthält.

4. Verfahren nach Anspruch 1, wobei das ausgewählte Medium Interleukin-3 enthält.

5. Verfahren nach Anspruch 1, wobei das ausgewählte Medium Granulocyten-Macrophagen Kolonie-stimulierenden Faktor enthält.

6. Verfahren nach Anspruch 1, wobei das ausgewählte Medium Granulocyten-Koloniestimulierenden Faktor enthält.

7. Verfahren nach Anspruch 1, wobei das ausgewählte Medium Interleukin-6 enthält.

8. Verfahren nach Anspruch 1, wobei das ausgewählte Medium Mastzellwachstumsfaktor enthält.

9. Verfahren nach Anspruch 1, wobei das ausgewählte Medium Interleukin-1 enthält.

10. Verfahren nach einem der Ansprüche 1 bis 9, wobei die CD34 positiven Zellen durch Immunselektion abgetrennt werden.

11. Verfahren nach Anspruch 10, wobei die CD34 positiven Zellen auf einer Affinitätssäule abgetrennt werden.

12. Verfahren nach Anspruch 10, wobei die CD34 positiven Zellen durch Flußcytometrie abgetrennt werden.

13. Verfahren nach einem der Ansprüche 1-12, wobei die CD34 positiven Zellen in einer Petrischale inkubiert werden.

14. Verfahren nach einem der Ansprüche 1-13, wobei die CD34 positiven Zellen in einem sterilen Beutel inkubiert werden.

15. Verfahren nach einem der Ansprüche 1-13, wobei die CD34 positiven Zellen in hohlen Fasern inkubiert werden.

16. Verfahren zur selektiven Expansion von CD34 positiven Zellen nach Anspruch 1, umfassend:
(a) Abtrennen von CD34 positiven Zellen von den anderen Zellen auf einer Affinitätssäule; und
(b) Inkubieren der abgetrennten CD34 positiven Zellen in einem sterilen Beutel mit einem Medium, das Stammzellwachstumsfaktor enthält.

## Revendications

1. Procédé de développement de manière sélective de cellules positives CD34, comprenant :
(a) la séparation de cellules positives CD34 d'autres cellules ; et
(b) l'incubation des cellules positives CD34 séparées dans un milieu sélectionné contenant un facteur de croissance de cellules souches, un facteur de croissance de mastocyte, l'Interleukine 2, l'Interleukine 3, l'Interleukine 4, l'Interleukine 6, l'Interleukine 7, G-CSF, GM-CSF, M-CSF, TGF-β, TNF-α, α-INF, FGF, PDGF, IGF-1 ou IGF-2, de sorte que les cellules positives CD34 sont augmentées de manière sélective en nombre.

2. Procédé selon la Revendication 1, pour développer de manière sélective des cellules positives CD34 comprenant :
(a) la séparation périodique de cellules positives CD34 de cellules matures; et
(b) l'incubation des cellules positives CD34 séparées dans ledit milieu sélectionné de sorte que les cellules positives CD34 sont augmentées de manière sélective en nombre.

3. Procédé selon la Revendication 1, dans lequel ledit milieu sélectionné contient un Facteur de Croissance de Cellules Souches.

4. Procédé selon la Revendication 1, dans lequel ledit milieu sélectionné contient de l'Interleukine 3.

5. Procédé selon la Revendication 1, dans lequel ledit milieu sélectionné contient un Facteur de Stimulation de Colonies de Macrophages-Granulocytes.

6. Procédé selon la Revendication 1, dans lequel ledit milieu sélectionné contient un Facteur de Stimulation de Colonies de Granulocytes.

7. Procédé selon la Revendication 1, dans lequel ledit milieu sélectionné contient de l'Interleukine 6.

8. Procédé selon la Revendication 1, dans lequel ledit milieu sélectionné contient un Facteur de Croissance de Mastocyte.

9. Procédé selon la Revendication 1, dans lequel ledit milieu sélectionné contient de l'Interleukine 1.

10. Procédé selon l'une quelconque des Revendications 1 à 9, dans lequel lesdites cellules positives CD34 sont séparées par immunosélection.

11. Procédé selon la Revendication 10, dans lequel lesdites cellules positives CD34 sont séparées sur une colonne d'affinité.

12. Procédé selon la Revendication 10, dans lequel lesdites cellules positives CD34 sont séparées par cytométrie en flux.

13. Procédé selon l'une quelconque des Revendications 1 à 12, dans lequel lesdites cellules positives CD34 séparées sont incubées dans une boîte de Pétri.

14. Procédé selon l'une quelconque des Revendications 1 à 13, dans lequel lesdites cellules positives CD34 séparées sont incubées dans un sac stérile.

15. Procédé selon l'une quelconque des Revendications 1 à 13, dans lequel lesdites cellules positives CD34 séparées sont incubées dans des fibres creuses.

16. Procédé selon la Revendication 1 pour développer de manière sélective des cellules positives CD34, comprenant :
(a) la séparation de cellules positives CD34 des autres cellules sur une colonne d'affinité; et
(b) l'incubation des cellules positives CD34 séparées dans un sac stérile avec un milieu contenant un Facteur de Croissance de Cellules Souches.
